# EUROPEAN PATENT SPECIFICATION

(11) **EP 1 098 660 B1**
(45) Date of publication and mention of the grant of the patent: **16.10.2002**
(21) Application number: 99933630.8
(22) Date of filing: 09.07.1999
(51) Int. Cl.: A61K 38/16, A61K 9/113, C07K 17/02, C07K 1/00

(54) **ENCAPSULATION OF WATER SOLUBLE PEPTIDES**
VERKAPSELUNG WASSERLÖSLICHER PEPTIDE
ENCAPSULATION DE PEPTIDES HYDROSOLUBLES

(30) Priority: 23.07.1998 US 121653; 23.07.1998 US 93914 P
(43) Date of publication of application: 16.05.2001
(60) Divisional application: 02076666.3
(73) Proprietor: SOCIETE DE CONSEILS DE RECHERCHES ET D'APPLICATIONS SCIENTIFIQUES S.A.S., 75016 Paris (FR)
(72) Inventor: IGNATIOUS, Francis, X., Millville, MA 01529 (US)
(74) Representative: Potter, Vanessa J.
(86) International application number: US9914869
(87) International publication number: WO00004916

(56) References cited:
- US-A- 5 160 745
- US-A- 5 445 832
- US-A- 5 578 709
- PATENT ABSTRACTS OF JAPAN, vol. 16, no. 207, 18 May 1992; & JP 04 036233 A (BIOMATERIAL UNIVERSE K.K.), 06 February 1992,

## Description

### Background of the Invention

This invention relates to a process for preparing biodegradable microspheres and or nanospheres using an oil-in-water process, which microspheres and nanospheres can be used for the controlled release of bioactive peptides.

A variety of techniques are described in the literature for the preparation of polymer microspheres for the sustained release of bioactive peptides. Among the different techniques such as spray drying, spray congealing, coacervation, solvent evaporation etc., solvent evaporation is simplest to scale-up industrially (for a recent review see protein delivery from biodegradable microspheres, by J.L. Cleland in Protein Delivery edited by L. Sanders and W. Hendren, Plenum Press, NY 1997). Solvent evaporation is usually practiced by dissolving or suspending an active ingredient in a polymer solution, which Is further dispersed in the form of droplets in a suitable medium containing surfactants capable of stabilizing the droplets, and the polymer droplets are hardened by evaporation of the solvent. When the polymer is dissolved in an organic medium and then emulsified in water, the process is called oil-in-water process (O/W). Water soluble peptides cannot be encapsulated by the O/W process, due to the partition of the water soluble peptides into the aqueous medium, resulting in low encapsulation efficiency. Higher encapsulation efficiencies were achieved by a more complex double emulsion water-in-oil-in-water (W/O/W) process (US Patent No. 5,271,945) or by using an oil-in-oil (O/O) process (EP 0330180 B1). The main drawback of the latter process is the use of different organic solvents, first to solubilize the polymer, and then to wash the polymer microspheres free of the oil in which they are formed. Therefore, the simple O/W emulsion solvent evaporation process is the most attractive, provided higher encapsulation efficiency can be achieved, since only one organic solvent is involved, and the residual organic solvent can be removed by vacuum drying.

The main hurdle to achieving higher encapsulation efficiency of the peptides is their water solubility. Solubility of peptides depends on the nature of the counter-ion. The aqueous solubility of a peptide is considerably reduced when the peptide is present as a free base, due to intermolecular interactions. One method of enhancing the encapsulation efficiency of the peptides in an O/W process according to the present invention, is by using a peptide as a free base adsorbed onto a bioresorbable inorganic matrix, such as hydroxyapatite, Calcium monohydrogen phosphate, zinc hydroxide, alum etc. In the case of encapsulation of LHRH agonists such as tryptorelin, leuprolin, goserlin, busrelin, etc., the presence of calcium phosphate in the micropheres may not only serve to stabilize the neutralized peptide but also act as a calcium supplement, since one of the biggest concerns of continuous therapy using LHRH agonists is loss of bone density. This method of encapsulation is most suited when the peptide loading in excess of 5-6% is not desired. In the case of high peptide loading, a heterogeneous distribution of the drug particles, even if they were stabilized by adsorption onto a solid matrix or not, inside the microspheres leads to non-predictable release profiles.

In cases where higher drug loading as well as predictable release profiles are desired, a second method of reducing the aqueous solubility of the drug, without sacrificing its potency, is by simply forming reversible water insoluble salts of mono-functional or multi-functional detergents and/or polymers or a combination of both, as exemplified by Schally et al. in US Patent No. 4,010,125. The aqueous solubility of the peptides can be considerably reduced by forming salts of mono-functional detergents such as sodium dodecyl sulfate, or of multi-functional anionic species such as pamoate, tannate, alginate, carboxymethyl cellulose, leading to the precipitation of the water insoluble peptide salt. Among the water insoluble salts, some exhibit good solubility in common organic solvents. U.S. Patent No. 5,672,659 describes compositions formed between anionic carboxylate functionalized polyesters and cationic peptides. These compositions as well as those formed with certain anionic detergents such as dioctylsulfosuccinate are found to exhibit good solubility in organic solvents such as dichloromethane (DCM), chloroform, acetonitrile, ethyl acetate, and the like.

During the water based encapsulation of the peptide, either as a free base adsorbed on to solid matrix or as water insoluble but organic solvent soluble salt, the pH of the aqueous medium can dramatically increase the water solubility, by affecting the equilibrium between the complexed and uncomplexed state. If the pH is not maintained at 7 the equilibrium may shift, favouring the solubilization of the peptide, leading to poor encapsulation efficiency.

It is therefore the object of the present invention to provide polymer microspheres and/or nanospheres prepared by a simple O/W method, where the encapsulation efficiency achieved can be greater than 85%.

### Summary of the Invention

In one aspect, the present invention is directed to a process for preparing polymer microspheres comprising a polymer and a peptide, which comprises the steps of:
neutralizing a peptide salt with a weak base in an aqueous medium wherein said medium comprises a suspension of hydroxyapatite or a solution of calcium mono-hydrogen phosphate to form a precipitate;
isolating the precipitate;
suspending the precipitate in an organic solvent, which comprises a polymer dissolved therein to form a suspension;
dispersing the suspension in an aqueous solution of a surfactant; and
evaporating the organic solvent to isolate the polymer microspheres.

A preferred process in accordance with the invention comprises the additional step of dissolving the peptide salt in a minimum of water before neutralizing the peptide salt.

A further preferred process in accordance with the invention is where the surfactant is one or more of sodium oleate, sodium stearate, sodium laurylsulphate, a poly(oxyethylene) sorbitan fatty acid ester, polyvinylpyrrolidine, polyvinyl alcohol, carboxymethyl cellulose, lecithin, geletin or hyaluronic acid.

A still further preferred process in accordance with the invention is where the surfactant is polyvinyl alcohol and the pH of the polyvinyl alcohol is 6.5-7.5.

A preferred process of any of the foregoing processes is where the pH of the polyvinyl alcohol is 6.9-7.1.

A preferred process of any of the foregoing processes is where the organic solvent is dichloromethane, chloroform or ethyl acetate.

A preferred process of any of the foregoing processes is where the organic solvent is dichloromethane and the concentration of the polymer in dichloromethane is 0.5% to 30% by weight.

A preferred process of any of the foregoing processes is where the concentration of the polymer in dichloromethane is 0.5% to 10% by weight.

A preferred process of any of the foregoing processes is where the peptide is growth hormone releasing peptide, luteinizing hormone-releasing hormone, somatostatin, bombesin, gastrin releasing peptide, calcitonin, bradykinin, galanin, melanocyte stimulating hormone, growth hormone releasing factor, amylin, tachykinins, secretin, parathyroid hormone, enkephalin, endothelin, calcitonin gene releasing peptide, neuromedins, parathyroid hormone related protein, glucagon, neurotensin, adrenocorticothrophic hormone, peptide YY, glucagon releasing peptide, vasoactive intestinal peptide, pituitary adenylate cyclase activating peptide, motilin, substance P, neuropeptide Y, or TSH or an analogue or a fragment thereof or a pharmaceutically acceptable salt thereof.

A preferred process of any of the foregoing processes is where the peptide is the LHRH analogue of the formula pyroGlu-His-Trp-Ser-Tyr-D-Trp-Leu-Arg-Pro-Gly-NH₂.

A preferred process of any of the foregoing processes is where the peptide is selected from the group of somatostatin analogues consisting of H-D-β-Nal-Cys-Tyr-D-Trp-Lys-Thr-Cys-Thr-NH₂,

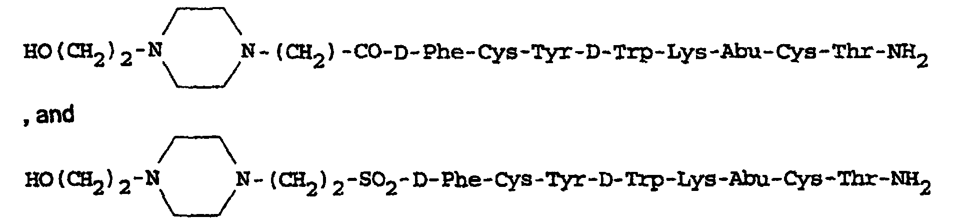

A preferred process of any of the foregoing processes is where the polymer is polylactide-co-glycolide, polycaprolactone or polyanhydride or a copolymer or blends thereof.

In another aspect, the present invention is directed to a polymer microsphere made according to the process of the first aspect of the invention.

Preferred of the immediately foregoing processes is where the polymer is polylactide-co-glycolide, polycaprolactone or polyanhydride or a copolymer or blends thereof and where the peptide is the LHRH analogue of the formula pyroGlu-His-Trp-Ser-Tyr-D-Trp-Leu-Arg-Pro-Gly-NH₂ or the peptide is selected from the group of somatostatin analogues consisting of H-D-β-Nal-Cys-Try-D-Trp-Lys-Thr-Cys-Thr-NH₂,

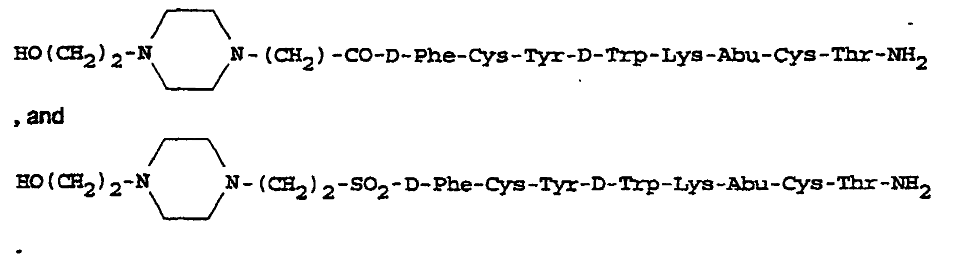

### Detailed Description of the Invention

The terms biodegradable and bioerodable are used interchangeably and is intended to mean that the material is degraded in the biological environment of the subject that to which it is administered.

Polymer microspheres made according to a process of this invention can be administered by intramuscular (IM), subcutaneous, pulmonary or oral route. Polymer nanospheres made according to a process of this invention in addition to being deliverable in the same manner as disclosed for microspheres can also be administered via inhalation methods such as those discussed in *Pulmonary Drug Delivery*, J. Yu and Y.W. Chien in Critical Reviews™ in Therapeutic Drug Carrier Systems, 14(4): 395-453, (1997). The microspheres and nanospheres made according to a process of this invention contain from less than 0.1% by weight up to approximately 50% by weight of a peptide. The polymer microspheres containing a peptide are prepared by an O/W emulsion solvent evaporation process, without compromising the much desired high encapsulation efficiency. Encapsulation efficiencies greater than 85% can be achieved according to the teachings of the present invention.

Polymers that can be used to form microspheres include bioerodible polymers such as polyesters (ex. polylactides, polyglycolides, polycaprolactone and copolymers and blends thereof), polycarbonates, polyorthoesters, polyacetals, polyanhydrides, their copolymers or blends, and non-bioerodible polymers such as polyacrylates, polystyrenes, polyvinylacetates, etc. Both types of polymers may optionally contain anionic or cationic groups. In general a polymer solution can be prepared containing between 1% and 20% polymer, preferably between 5% and 15% polymer. The polymer solution can be prepared in dichloromethane (DCM), chloroform, ethylacetate, methylformate, dichloroethane, toluene, cyclohexane and the like.

Any peptide can be incorporated in the microspheres of this invention. Examples of peptides that can be incorporated in the microspheres produced by a process of this invention are growth hormone releasing peptide (GHRP), luteinizing hormone-releasing hormone (LHRH), somatostatin, bombesin, gastrin releasing peptide (GRP), calcitonin, bradykinin, galanin, melanocyte stimulating hormone (MSH), growth hormone releasing factor (GRF), amylin, tachykinins, secretin, parathyroid hormone (PTH), enkephalin, endothelin, calcitonin gene releasing peptide (CGRP), neuromedins, parathyroid hormone related protein (PTHrP), glucagon, neurotensin, adrenocorticothrophic hormone (ACTH), peptide YY (PYY), glucagon releasing peptide (GLP), vasoactive intestinal peptide (VIP), pituitary adenylate cyclase activating peptide (PACAP), motilin, substance P, neuropeptide Y (NPY), TSH and analogs and fragments thereof or a pharmaceutically acceptable salt thereof.

The term "peptide" is intended to include peptide, polypeptides and proteins.

Examples of specific LHRH analogues that can be incorporated in the microspheres of this invention are tryptorelin (p-Glu-His-Trp-Ser-Tyr-D-Trp-Leu-Arg-Pro-Gly-NH₂), buserelin ([D-Ser(t-Bu)⁶, des-Gly-NH₂¹⁰]-LHRH(1-9)NHEt), deslorelin ([D-Trp⁶, des-Gly-NH₂¹⁰]-LHRH(1-9)NHEt, fertirelin ([des-Gly-NH₂¹⁰]-LHRH(1-9)NHEt), gosrelin ([D-Ser(t-Bu)⁶, Azgly¹⁰]-LHRH), histrelin ([D-His(Bzl)⁶, des-Gly-NH₂¹⁰]-LHRH(1-9)NHEt), leuprorelin ([D-Leu⁶, des-Gly-NH₂¹⁰]-LHRH(1-9)NHEt), lutrelin ([D-Trp⁶, MeLeu⁷, des-Gly-NH₂¹⁰]-LHRH(1-9)NHEt), nafarelin ([D-Nal⁶]-LHRH and pharmaceutically acceptable salts thereof.

Preferred somatostatin analogs that can be incorporated in the microspheres and/or nanospheres of this invention are those covered by formulae or those specifically recited in the publications set forth below, all of which are hereby incorporated by reference:
Van Binst, G. et al. Peptide Research 5:8 (1992);
Horvath, A. et al. Abstract, "Conformations of Somatostatin Analogs Having Antitumor Activity", 22nd European peptide Symposium, September 13-19, 1992, Interlaken, Switzerland;
PCT Application WO 91/09056 (1991);
EP Application 0 363 589 A2 (1990);
U.S. Patent No. 4,904,642 (1990);
U.S. Patent No. 4,871,717 (1989);
U.S. Patent No. 4,853,371 (1989);
U.S. Patent No. 4,725,577 (1988);
U.S. Patent No. 4,684,620 (1987)
U.S. Patent No. 4,650,787 (1987);
U.S. Patent No. 4,603,120 (1986);
U.S. Patent No. 4,585,755 (1986);
EP Application 0 203 031 A2 (1986);
U.S. Patent No. 4,522,813 (1985);
U.S. Patent No. 4,486,415 (1984);
U.S. Patent No. 4,485,101 (1984);
U.S. Patent No. 4,435,385 (1984);
U.S. Patent No. 4,395,403 (1983);
U.S. Patent No. 4,369,179 (1983);
U.S. Patent No. 4,360,516 (1982);
U.S. Patent No. 4,358,439 (1982);
U.S. Patent No. 4,328,214 (1982);
U.S. Patent No. 4,316,890 (1982);
U.S. Patent No. 4,310,518 (1982);
U.S. Patent No. 4,291,022 (1981);
U.S. Patent No. 4,238,481 (1980);
U.S. Patent No. 4,235,886 (1980);
U.S. Patent No. 4,224,190 (1980);
U.S. Patent No. 4,211,693 (1980);
U.S. Patent No. 4,190,648 (1980);
U.S. Patent No. 4,146,612 (1979);
U.S. Patent No. 4,133,782 (1979);
U.S. Patent No. 5,506,339 (1996);
U.S. Patent No. 4,261,885 (1981);
U.S. Patent No. 4,728,638 (1988);
U.S. Patent No. 4,282,143 (1981);
U.S. Patent No. 4,215,039 (1980);
U.S. Patent No. 4,209,426 (1980);
U.S. Patent No. 4,190,575 (1980);
EP Patent No. 0 389 180 (1990);
EP Application No. 0 505 680 (1982);
EP Application No. 0 083 305 (1982);
EP Application No. 0 030 920 (1980);
PCT Application No. WO 88/05052 (1988);
PCT Application No. WO 90/12811 (1990);
PCT Application No. WO 97/01579 (1997);
PCT Application No. WO 91/18016 (1991);
U.K. Application No. GB 2,095,261 (1981); and
French Application No. FR 2,522,655 (1983).

Examples of somatostatin analogs include, but are not limited to, the following somatostatin analogs which are disclosed in the above-cited references:
H-D-β-Nal-Cys-Tyr-D-Trp-Lys-Thr-Cys-Thr-NH₂;
H-D-Phe-Cys-Phe-D-Trp-Lys-Thr-Cys-β-Nal-NH₂;
H-D-Phe-Cys-Tyr-D-Trp-Lys-Thr-Cys-β-Nal-NH₂;
H-D-β-Nal-Cys-Phe-D-Trp-Lys-Thr-Cys-Thr-NH₂;
H-D-Phe-Cys-Tyr-D-Trp-Lys-Thr-Pen-Thr-NH₂;
H-D-Phe-Cys-Phe-D-Trp-Lys-Thr-Pen-Thr-NH₂;
H-D-Phe-Cys-Tyr-D-Trp-Lys-Thr-Pen-Thr-OH;
H-D-Phe-Cys-Phe-D-Trp-Lys-Thr-Pen-Thr-OH;
H-Gly-Pen-Phe-D-Trp-Lys-Thr-Cys-Thr-OH;
H-Phe-Pen-Tyr-D-Trp-Lys-Thr-Cys-Thr-OH;
H-Phe-Pen-Phe-D-Trp-Lys-Thr-Pen-Thr-OH;
H-D-Phe-Cys-Phe-D-Trp-Lys-Thr-Cys-Thr-ol;
H-D-Phe-Cys-Phe-D-Trp-Lys-Thr-Cys-Thr-NH₂;
H-D-Trp-Cys-Tyr-D-Trp-Lys-Val-Cys-Thr-NH₂;
H-D-Trp-Cys-Phe-D-Trp-Lys-Thr-Cys-Thr-NH₂;
H-D-Phe-Cys-Tyr-D-Trp-Lys-Val-Cys-Thr-NH₂;
H-D-Phe-Cys-Tyr-D-Trp-Lys-Val-Cys-Trp-NH₂;
H-D-Phe-Cys-Tyr-D-Trp-Lys-Val-Cys-Thr-NH₂;
Ac-D-Phe-Lys*-Tyr-D-Trp-Lys-Val-Asp*-Thr-NH₂ (an amide bridge formed between Lys* and Asp*);
Ac-hArg(Et)₂-Gly-Cys-Phe-D-Trp-Lys-Thr-Cys-Thr-NH₂;
Ac-D-hArg(Et)₂-Gly-Cys-Phe-D-Trp-Lys-Thr-Cys-Thr-NH₂;
Ac-D-hArg(Bu)-Gly-Cys-Phe-D-Trp-Lys-Thr-Cys-Thr-NH₂;
Ac-D-hArg(Et)₂-Cys-Phe-D-Trp-Lys-Thr-Cys-Thr-NH₂;
Ac-L-hArg(Et)₂-Cys-Phe-D-Trp-Lys-Thr-Cys-Thr-NH₂;
Ac-D-hArg(CH₂CF₃)₂-Cys-Phe-D-Trp-Lys-Thr-Cys-Thr-NH₂;
Ac-D-hArg(CH₂CF₃)₂-Gly-Cys-Phe-D-Trp-Lys-Thr-Cys-Thr-NH₂;
Ac-D-hArg(CH₂CF₃)₂-Gly-Cys-Phe-D-Trp-Lys-Thr-Cys-Phe-NH₂;
Ac-D-hArg(CH₂CF₃)₂-Gly-Cys-Phe-D-Trp-Lys-Thr-Cys-Thr-NHEt;
Ac-L-hArg(CH₂-CF₃)₂-Gly-Cys-Phe-D-Trp-Lys-Thr-Cys-Thr-NH₂;
Ac-D-hArg(CH₂CF₃)₂-Gly-Cys-Phe-D-Trp-Lys(Me)-Thr-Cys-Thr-NH₂;
Ac-D-hArg(CH₂CF₃)₂-Gly-Cys-Phe-D-Trp-Lys(Me)-Thr-Cys-Thr-NHEt;
Ac-hArg(CH₃, hexyl)-Gly-Cys-Phe-D-Trp-Lys-Thr-Cys-Thr-NH₂;
H-hArg(hexyl₂)-Gly-Cys-Phe-D-Trp-Lys-Thr-Cys-Thr-NH₂;
Ac-D-hArg(Et)₂-Gly-Cys-Phe-D-Trp-Lys-Thr-Cys-Thr-NHEt;
Ac-D-hArg(Et)₂-Gly-Cys-Phe-D-Trp-Lys-Thr-Cys-Phe-NH₂;
Propionyl-D-hArg(Et)₂-Gly-Cys-Phe-D-Trp-Lys(iPr)-Thr-Cys-Thr-NH₂;
Ac-D-β-Nal-Gly-Cys-Phe-D-Trp-Lys-Thr-Cys-Gly-hArg(Et)₂-NH₂;
Ac-D-Lys(iPr)-Gly-Cys-Phe-D-Trp-Lys-Thr-Cys-Thr-NH₂;
Ac-D-hArg(CH₂CF₃)₂-D-hArg(CH₂CF₃)₂-Gly-Cys-Phe-D-Trp-Lys-Thr-Cys-Thr-NN₂;
Ac-D-hArg(CH₂CF₃)₂-D-hArg(CH₂CF₃)₂-Gly-Cys-Phe-D-Trp-Lys-Thr-Cys-Phe-NH₂;
Ac-D-hArg(Et)₂-D-hArg(Et)₂-Gly-Cys-Phe-D-Trp-Lys-Thr-Cys-Thr-NH₂;
Ac-Cys-Lys-Asn-4-Cl-Phe-Phe-D-Trp-Lys-Thr-Phe-Thr-Ser-D-Cys-NH₂;
H-Bmp-Tyr-D-Trp-Lys-Val-Cys-Thr-NH₂;
H-Bmp-Tyr-D-Trp-Lys-Val-Cys-Phe-NH₂;
H-Bmp-Tyr-D-Trp-Lys-Val-Cys-p-Cl-Phe-NH₂;
H-Bmp-Tyr-D-Trp-Lys-Val-Cys-β-Nal-NH₂;
H-D-β-Nal-Cys-Tyr-D-Trp-Lys-Val-Cys-Thr-NH₂;
H-D-Phe-Cys-Tyr-D-Trp-Lys-Abu-Cys-Thr-NH₂;
H-D-Phe-Cys-Tyr-D-Trp-Lys-Abu-Cys-β-Nal-NH₂;
H-pentafluoro-D-Phe-Cys-Tyr-D-Trp-Lys-Val-Cys-Thr-NH₂;
Ac-D-β-Nal-Cys-pentafluoro-Phe-D-Trp-Lys-Val-Cys-Thr-NH₂;
H-D-β-Nal-Cys-Tyr-D-Trp-Lys-Val-Cys-β-Nal-NH₂;
H-D-Phe-Cys-Tyr-D-Trp-Lys-Val-Cys-β-Nal-NH₂;
H-D-β-Nal-Cys-Tyr-D-Trp-Lys-Abu-Cys-Thr-NH₂;
H-D-p-Cl-Phe-Cys-Tyr-D-Trp-Lys-Abu-Cys-Thr-NH₂;
Ac-D-p-Cl-Phe-Cys-Tyr-D-Trp-Lys-Abu-Cys-Thr-NH₂;
H-D-Phe-Cys-β-Nal-D-Trp-Lys-Val-Cys-Thr-NH₂;
H-D-Phe-Cys-Tyr-D-Trp-Lys-Cys-Thr-NH₂;
cyclo(Pro-Phe-D-Trp-N-Me-Lys-Thr-Phe);
cydo(Pro-Phe-D-Trp-N-Me-Lys-Thr-Phe);
cyclo(Pro-Phe-D-Trp-Lys-Thr-N-Me-Phe);
cyclo(N-Me-Ala-Tyr-D-Trp-Lys-Thr-Phe);
cyclo(Pro-Tyr-D-Trp-Lys-Thr-Phe);
cyclo(Pro-Phe-D-Trp-Lys-Thr-Phe);
cyclo(Pro-Phe-L-Trp-Lys-Thr-Phe);
cydo(Pro-Phe-D-Trp(F)-Lys-Thr-Phe);
cyclo(Pro-Phe-Trp(F)-Lys-Thr-Phe);
cyclo(Pro-Phe-D-Trp-Lys-Ser-Phe);
cyclo(Pro-Phe-D-Trp-Lys-Thr-p-Cl-Phe);
cyclo(D-Ala-N-Me-D-Phe-D-Thr-D-Lys-Trp-D-Phe);
cyclo(D-Ala-N-Me-D-Phe-D-Val-Lys-D-Trp-D-Phe);
cyclo(D-Ala-N-Me-D-Phe-D-Thr-Lys-D-Trp-D-Phe);
cyclo(D-Abu-N-Me-D-Phe-D-Val-Lys-D-Trp-D-Tyr);
cyclo(Pro-Tyr-D-Trp-t-4-AchxAla-Thr-Phe);
cyclo(Pro-Phe-D-Trp-t-4-AchxAla-Thr-Phe);
cyclo(N-Me-Ala-Tyr-D-Trp-Lys-Val-Phe);
cyclo(N-Me-Ala-Tyr-D-Trp-t-4-AchxAla-Thr-Phe);
cyclo(Pro-Tyr-D-Trp-4-Amphe-Thr-Phe);
cyclo(Pro-Phe-D-Trp-4-Amphe-Thr-Phe);
cyclo(N-Me-Ala-Tyr-D-Trp-4-Amphe-Thr-Phe);
cyclo(Asn-Phe-Phe-D-Trp-Lys-Thr-Phe-Gaba);
cyclo(Asn-Phe-Phe-D-Trp-Lys-Thr-Phe-Gaba-Gaba);
cyclo(Asn-Phe-D-Trp-Lys-Thr-Phe);
cyclo(Asn-Phe-Phe-D-Trp-Lys-Thr-Phe-NH(CH₂)₄CO);
cyclo(Asn-Phe-Phe-D-Trp-Lys-Thr-Phe-β-Ala);
cyclo(Asn-Phe-Phe-D-Trp-Lys-Thr-Phe-D-Glu)-OH;
cyclo(Phe-Phe-D-Trp-Lys-Thr-Phe);
cyclo(Phe-Phe-D-Trp-Lys-Thr-Phe-Gly);
cyclo(Phe-Phe-D-Trp-Lys-Thr-Phe-Gaba);
cyclo(Asn-Phe-Phe-D-Trp-Lys-Thr-Phe-Gly);
cyclo(Asn-Phe-Phe-D-Trp(F)-Lys-Thr-Phe-Gaba);
cyclo(Asn-Phe-Phe-D-Trp(NO₂)-Lys-Thr-Phe-Gaba);
cyclo(Asn-Phe-Phe-Trp(Br)-Lys-Thr-Phe-Gaba);
cyclo(Asn-Phe-Phe-D-Trp-Lys-Thr-Phe(I)-Gaba);
cyclo(Asn-Phe-Phe-D-Trp-Lys-Thr-Tyr(But)-Gaba);
cyclo(Bmp-Lys-Asn-Phe-Phe-D-Trp-Lys-Thr-Phe-Thr-Pro-Cys)-OH;
cyclo(Bmp-Lys-Asn-Phe-Phe-D-Trp-Lys-Thr-Phe-Thr-Pro-Cys)-OH;
cyclo(Bmp-Lys-Asn-Phe-Phe-D-Trp-Lys-Thr-Phe-Thr-Tpo-Cys)-OH;
cyclo(Bmp-Lys-Asn-Phe-Phe-D-Trp-Lys-Thr-Phe-Thr-MeLeu-Cys)-OH;
cyclo(Phe-Phe-D-Trp-Lys-Thr-Phe-Phe-Gaba);
cyclo(Phe-Phe-D-Trp-Lys-Thr-Phe-D-Phe-Gaba);
cyclo(Phe-Phe-D-Trp(5F)-Lys-Thr-Phe-Phe-Gaba);
cyclo(Asn-Phe-Phe-D-Trp-Lys(Ac)-Thr-Phe-NH-(CH₂)₃-CO);
cyclo(Lys-Phe-Phe-D-Trp-Lys-Thr-Phe-Gaba);
cyclo(Lys-Phe-Phe-D-Trp-Lys-Thr-Phe-Gaba);
cycle(Orn-Phe-Phe-D-Trp-Lys-Thr-Phe-Gaba);
H-Cys-Phe-Phe-D-Trp-Lys-Thr-Phe-Cys-NH₂;
H-Cys-Phe-Phe-D-Trp-Lys-Ser-Phe-Cys-NH₂;
H-Cys-Phe-Tyr-D-Trp-Lys-Thr-Phe-Cys-NH₂; and
H-Cys-Phe-Tyr(I)-D-Trp-Lys-Thr-Phe-Cys-NH₂.

A disulfide bridge is formed between the two free thiols (e.g., Cys, Pen, or Bmp residues) when they are present in a peptide; however, the disulfide bond is not shown.

Also included are somatostatin agonists of the following formula: 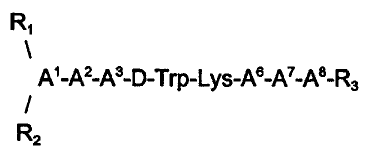 wherein
A¹ is a D- or L- isomer of Ala, Leu, lle, Val, Nle, Thr, Ser, β-Nal, β-Pal, Trp, Phe, 2,4-dichloro-Phe, pentafluoro-Phe, p-X-Phe, or o-X-Phe, wherein X is CH₃, Cl, Br, F, OH, OCH₃ or NO₂;
A² is Ala, Leu, lie, Val, Nle, Phe, β-Nal, pyridyl-Ala, Trp, 2,4-dichloro-Phe, pentafluoro-Phe, o-X-Phe, or p-X-Phe, wherein X is CH₃, Cl, Br, F, OH, OCH₃ or NO₂;
A³ is pyridyl-Ala, Trp, Phe, β-Nal, 2,4-dichloro-Phe, pentafluoro-Phe, o-X-Phe, or p-X-Phe, wherein X is CH₃, Cl, Br, F, OH, OCH₃ or NO₂;
A⁶ is Val, Ala, Leu, lle, Nle, Thr, Abu, or Ser;
A⁷ is Ala, Leu, lle, Val, Nle, Phe, β-Nal, pyridyl-Ala, Trp, 2,4-dichloro-Phe, pentafluoro-Phe, o-X-Phe, or p-X-Phe, wherein X is CH₃, Cl, Br, F, OH, OCH₃ or NO₂;
A⁸ is a D- or L-isomer of Ala, Leu, Ile, Val, Nle, Thr, Ser, Phe, β-Nal, pyridyl-Ala, Trp, 2,4-dichloro-Phe, pentafluoro-Phe, p-X-Phe, or o-X-Phe, wherein X is CH₃, Cl, Br, F, OH, OCH₃ or NO₂;
each R₁ and R₂, independently, is H, lower acyl or lower alkyl; and R₃ is OH or NH₂; provided that at least one of A¹ and A⁸ and one of A² and A⁷ must be an aromatic amino acid; and further provided that A¹, A², A⁷ and A⁸ cannot all be aromatic amino acids.

Examples of linear agonists to be used in a process of this invention include:
H-D-Phe-p-chloro-Phe-Tyr-D-Trp-Lys-Thr-Phe-Thr-NH₂;
H-D-Phe-p-NO₂-Phe-Tyr-D-Trp-Lys-Val-Phe-Thr-NH₂;
H-D-Nal-p-chloro-Phe-Tyr-D-Trp-Lys-Val-Phe-Thr-NH₂;
H-D-Phe-Phe-Phe-D-Trp-Lys-Thr-Phe-Thr-NH₂;
H-D-Phe-Phe-Tyr-D-Trp-Lys-Val-Phe-Thr-NH₂;
H-D-Phe-p-chloro-Phe-Tyr-D-Trp-Lys-Val-Phe-Thr-NH₂; and
H-D-Phe-Ala-Tyr-D-Trp-Lys-Val-Ala-β-D-Nal-NH₂.

If desired, one or more chemical moieties, e.g., a sugar derivative, mono or poly-hydroxy C₂₋₁₂ alkyl, mono or poly-hydroxy C₂₋₁₂ acyl groups, or a piperazine derivative, can be attached to the somatostatin agonist, e.g., to the N-terminus amino acid. See PCT Application WO 88/02756, European Application 0 329 295, and PCT Application No. WO 94/04752. An example of somatostatin agonists which contain N-terminal chemical substitutions are:

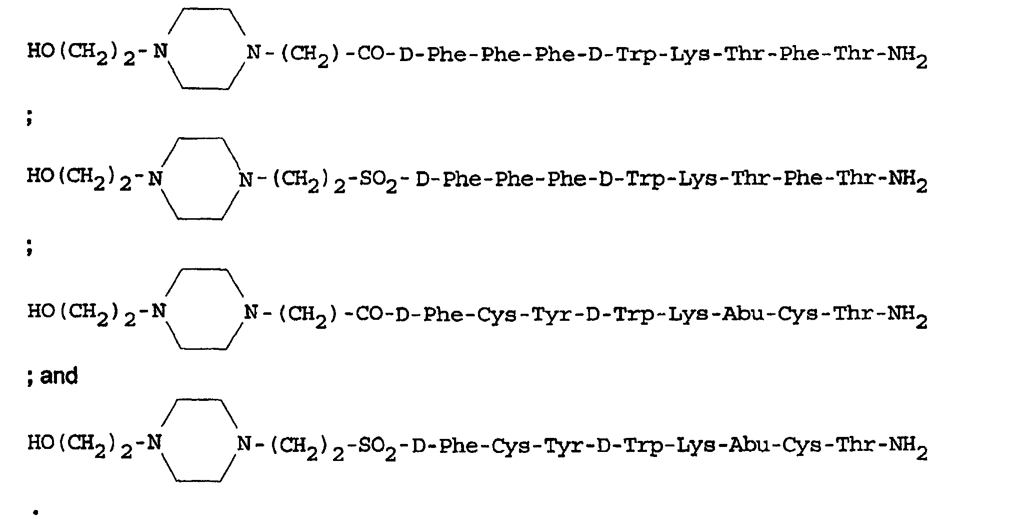

Processes for making polymer microspheres and/or nanospheres according to a method of the present invention are described herein. The examples are given for illustrative purposes and are not meant to limit the scope of the present invention. All references cited herein are incorporated herein by reference.

Water solubility can be considerably diminished by co-precipitating the peptide as free base along with an inorganic bioresorbable matrix such as hydroxyapatite, calcium phosphate, alum, zinc hydroxide, etc. The presence of the inorganic bioresorbable matrix stabilizes the free, neutralized peptide by a combination of phenomena such as complexation, adsorption and the like.

The water insoluble peptide in the neutralized and adsorbed form can be prepared by dissolving a water soluble salt of a peptide such as acetate, trifluoroacetate, hydrochloride, sulphate, and the like, in a minimum amount of water and suspending hydroxyapatite in the solution, followed by addition of a weak base such as NaHCO₃, triethylamine, and the like to bring the pH up to 7-8. The precipitate so formed is filtered, suspended in water and lyophilized.

Polymers that can be used to form microspheres include biodegradable polymers such as polyester (ex. polyactides, polyglycolides, polycaprolactone and copolymers and blends thereof) polycarbonates, polyorthoesters, polyacetals, polyanhydrides, their copolymers or blends, and non-biodegradable polymers such as polyacrylates, polystyrenes, polyvinylacetates, etc. the biodegradable polymers are intended to degrade under physiological conditions over a period of time, to yield natural metabolites, such that the implant or the depot does not require to be retrieved once the drug is exhausted. These polymers may optionally contain anionic or cationic groups. The anionic groups present in the polymer may be sulphate, phosphate, or carboxylate, capable of forming salts with basic bioactive substances. The polymers can be endowed with cationic functionalities (or basic groups), such as amino, amidino, guadino, ammonium, cyclic amino groups and nucleic acid bases, which can form salts with acidic bioactive molecules. In general a polymer solution can be prepared in a water immiscible organic solvent, containing between 1% and 20% polymer, preferably between 5% and 15%.

The polymer solution can be prepared in water immiscible organic solvents such as dichloromethane (DCM), chloroform, dichloroethane, trichloroethane, cyclohexane, benzene, toluene, ethyl acetate, and the like, which can be used alone or as a mixture thereof.

The polymer microspheres of the invention are made by either suspending or dissolving the coprecipitates, salts or complexes in a polymer solution, and emulsifying this mixture/solution in aqueous medium containing a surfactant.

Emulsification of the oil droplets in aqueous medium is performed by known methods of dispersion. The dispersion methods include the use of mixers such as propeller mixer, turbine mixer, colloid mill method, the homogenizer method, and the ultrasonic irradiation method.

The emulsification of the organic layer is done in an aqueous layer containing an emulsifier, which can stabilize O/W emulsions, such as anionic surfactants (sodium oleate, sodium stearate, sodium laurylsulphate, and the like), non-ionic surfactants such as poly(oxyethylene) sorbitan fatty acid esters like Tween 20®, Tween 60®, Tween 80®, polyvinylpyrrolidone, polyvinyl alcohol, carboxymethyl cellulose, lecithin, gelatin, hyaluronic acid and the like, which may be used separately or in combination. The amount used may be chosen appropriately from a range of about 0.01% to 20%, preferably about 0.05% to 10%.

One important aspect of the present invention is the role of the pH of the aqueous surfactant medium in which the emulsion droplets are formed, in partitioning the peptide into the aqueous medium, thereby reducing the encapsulation efficiency. Encapsulation efficiency is the amount of peptide actually present in the microspheres compared to the amount initially used in the process. The peptide loss to the aqueous medium can be minimized by maintaining the pH of the aqueous medium between 6-8, preferably around 7.

Removal of the solvent in the oil phase is performed by any method known in the art: solvent removal may be effected by gradual reduction of pressure by stirring with a propeller type stirrer or a magnetic stirrer, or by adjusting the degree of vacuum with a rotary evaporator.

Microspheres and/or nanospheres formed by the removal of the solvent are collected by centrifugation or by filtration, followed by several repetitions of washing with deionized water to remove emulsifier and any unencapsulated peptide.

The washed microspheres are collected by filtration and dried under vacuum at about 30° C for about 24-48 hrs., in order to remove the residual solvent.

The peptide content of a microspheres and/or nanospheres made according to a process of this was determined by nitrogen analysis and also by HPLC method. In the HPLC method, about 20 mg of the sample dissolved in 0.1% TFA solution, was analyzed using a C₁₈ column, using eluants A (0.1% TFA) and eluant B (80% acetonitrile, 0.1% TFA), programmed at a gradient of 20% to 80% B in 50 min, and the peptide was monitored at 280nm by a UV detector (Applied Biosystems, Model # 785A). The HPLC system consisted of two Waters 510 pumps, Waters automated gradient controller and a Waters 712 wisp (Waters, Milford, MA).

### Example 1

### 1(a): Preparation of neutralized Tryptorelin in presence of hydroxylapatite

200 mg of Hydroxyapatite (HAP) (American International Chemical, Natick, MA having particle size 2 µm) was suspended in water. 100 mg of the acetate salt of pyroGlu-His-Trp-Ser-Tyr-D-Trp-Leu-Arg-Pro-Gly-NH₂ (Tryptorelin, Kinerton, Dublin, Ireland) was dissolved in 1 ml of water and this solution was added to the suspension of HAP. The pH of the slurry was brought to about 7-8 by adding 1N NₐHCO₃ dropwise. The precipitate was left stirring for about 2 hrs. The precipitate was collected by centrifugation. The precipitate was suspended in water and lyophilized.

Peptide content by nitrogen analysis = 23.6% and by HPLC=22.1%.

### 1(b): Preparation of neutralized polyvinyl alcohol (PVA) solution

Commercially available PVA has pH lower than 5, due to the presence of hydrolysis product of poly(vinylacetate) from which PVA is prepared. The PVA solution was cleaned by preparing a concentrated solution in water, neutralized with NaHCO₃ solution, dialyzing against de-ionized water. The neutralized PVA was precipitated in acetone, filtered and vacuum dried.

### 1(c): Preparation of p(d1-lactic acid) microspheres

1 g of p(dl-lactic acid) available from (Pharma-Biotech. ZI de Signes, BP 707, 83030 Toulon Cedex-9, France) (Mn=32K, Mw=54.4K) was dissolved in 10 ml DCM and 100 mg of the example 1(a) was suspended in the solution. The solution was cooled in an ice-bath and was dispersed in 100 ml of 1% pre-cooled PVA (polyvinyl alcohol) solution using a Polytron homogenizer (Kinematica, Switzerland). DCM was rotovaped and the microspheres were collected by centrifugation. The particles were suspended in water and lyophilized. Peptide content. determined by nitrogen analysis was 2% (calculated 2.2%).

### 1(d): Preperation of neutralized Tryptorelin in presence of HAP

To 500 mg of acetate salt of pyroGlu-His-Trp-Ser-Tyr-DTrp-Leu-Arg-Pro-Gly-NH₂ (Kinerton, Dublin, Ireland) dissolved in 5 ml of water was added 200 mg of HAP. The pH of the solution was brought up to 7-8 using 1N NaHCO₃. The solution was left standing for about 2 hrs. and the precipitate was collected by centrifugation, and suspended in water and lyophilized. Peptide content by nitrogen analysis = 58.9%.

### 1(e): Preparation of microspheres containing 1(d)

Microspheres were prepared by employing the same procedure as 1(b) and 1(c).
Peptide content 4.9%.

### 1(f): Co-precipitation of Tryptorelin and Calcium Phophate monobasic

A solution of 100 mg of CaRPO₄ (Aldrich Chemicals, St. Louis, MO) and 100 mg of the acetate salt of pyroGlu-His-Trp-Ser-Tyr-D-Trp-Leu-Arg-Pro-Gly-NH₂ (Kinerton, Dublin, Ireland) in water was prepared. The pH of the solution was brought to about 7 using 1N NaHCO₃ and left for about 24 hrs. for the completion of the precipitate. The precipitate was centrifuged, collected, suspended in water and lyophilized. Peptide content determined by HPLC method was 49.4%.

### 1(g): In-Vivo testing of 1(c)and 1(e) in rats

Formulations 1(c)& 1(e) were administered in male rats by IM injection at a dose of 300 µg of tryptorelin equivalent per rat, as a dispersion of the microspheres in 1% (w/v) Tween 20® (Aldrich Chemicals, St. Louis. MO) and 2% (w/v) carboxymethyl cellulose (Aldrich Chemicals, St. Louis, MO). The testosterone response was monitored by RIA: 50µL of the blood sample, 200µL of 1251-testosterone and 200µL of antiserum were poured into tubes which were shaken and incubated for 2 hrs. at 37°C. The Immunoprecipitant reagent (1ml) was added to each tube and all the tubes were incubated for 15 minutes at room temperature. The supernatent was eliminated after centrifugation and the radioactivity was measured with LKB Wallace gamma counter. The plasma testosterone levels are shown below.

**Table 1**

| Plasma testosterone response (ng/ml) to IM injection of 300 µg of Tryptorelin equivalent/rat. | | | | | | | | | |
|---|---|---|---|---|---|---|---|---|---|
| Sample | 6h | Day 2 | Day 3 | Day 5 | Day 10 | Day 15 | Day 23 | Day 30 | Day 37 |
| 1(c) | 5.37 | 4.09 | 0.74 | 0.45 | 0.30 | 0.31 | 0.90 | 0.61 | 0.81 |
| 1(e) | 5.32 | 3.58 | 1.04 | 0.29 | 0.38 | 0.56 | 0.80 | 0.75 | 0.72 |

## Claims

1. A process for preparing polymer microspheres comprising a polymer and a peptide, which comprises the steps of:
neutralizing a peptide salt with a weak base in an aqueous medium wherein said medium comprises a suspension of hydroxyapatite or a solution of calcium mono-hydrogen phosphate to form a precipitate;
isolating the precipitate;
suspending the precipitate in an organic solvent, which comprises a polymer dissolved therein to form a suspension;
dispersing the suspension in an aqueous solution of a surfactant; and
evaporating the organic solvent to isolate the polymer microspheres.

2. A process according to claim 1, comprising the additional step of dissolving the peptide salt in a minimum of water before neutralizing the peptide salt.

3. A process according to claim 2, wherein the surfactant is one or more of sodium oleate, sodium stearate, sodium laurylsulphate, a poly(oxyethylene) sorbitan fatty acid ester, polyvinylpyrrolidine, polyvinyl alcohol, carboxymethyl cellulose, lecithin, gelatin or hyaluronic acid.

4. A process according to claim 3, wherein the surfactant is polyvinyl alcohol and the pH of the polyvinyl alcohol is 6.5-7.5.

5. A process according to claim 4, wherein the pH of the polyvinyl alcohol is 6.9-7.1.

6. A process according to claim 5, wherein the organic solvent is dichloromethane, chloroform or ethyl acetate.

7. A process according to claim 6, wherein the organic solvent is dichloromethane and the concentration of the polymer in the organic solvent is 0.5% to 30% by weight.

8. A process according to claim 7, wherein the concentration of the polymer in dichloromethane is 0.5% to 10% by weight.

9. A process according to claim 8, wherein the peptide is growth hormone releasing peptide, luteinizing hormone-releasing hormone, somatostatin, bombesin, gastrin releasing peptide, calcitonin, bradykinin, galanin, melanocyte stimulating hormone, growth hormone releasing factor, amylin, tachykinins, secretin, parathyroid hormone, enkephalin, endothelin, calcitonin gene releasing peptide, neuromedins, parathyroid hormone related protein, glucagon, neurotensin, adrenocorticothrophic hormone, peptide YY, glucagon releasing peptide, vasoactive intestinal peptide, pituitary adenylate cyclase activating peptide, motilin, substance P, neuropeptide Y, or TSH or an analogue or a fragment thereof or a pharmaceutically acceptable salt thereof.

10. A process according to claim 9, wherein the peptide is the LHRH analogue of the formula pyroGlu-His-Trp-Ser-Tyr-D-Trp-Leu-Arg-Pro-Gly-NH₂.

11. A process according to claim 10, wherein the polymer is polylactide-co-glycolide, polycaprolactone or polyanhydride or a copolymer or blends thereof.

12. A process according to claim 9, wherein the peptide is selected from the group of somatostatin analogues consisting of H-D-β-Nal-Cys-Tyr-D-Trp-Lys-Thr-Cys-Thr-NH₂,

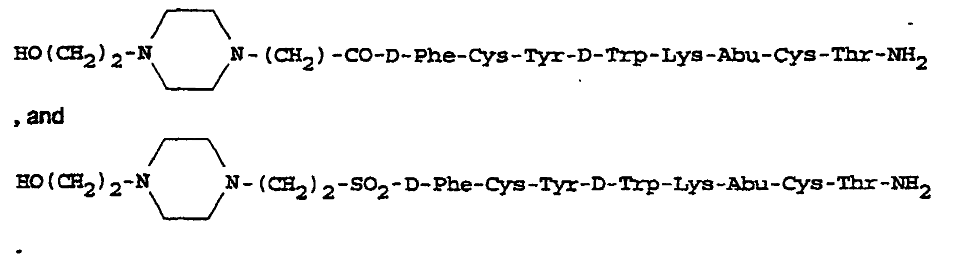

13. A process according to claim 12, wherein the polymer is polylactide-co-glycolide, polycaprolactone or polyanhydride or a copolymer or blends thereof.

14. A polymer microsphere obtainable by the process of claim 1.

15. A polymer microsphere obtainable by the process of claim 11.

16. A polymer microsphere obtainable by the process of claim 13

## Patentansprüche

1. Verfahren zur Herstellung von Polymermikrosphären, die ein Polymer und ein Peptid umfassen, das folgende Schritte umfasst:
Neutralisieren eines Peptidsalzes mit einer schwachen Base in einem wässrigen Medium, wobei das Medium eine Suspension von Hydroxyapatit oder eine Lösung von Calciummonohydrogenphosphat zur Bildung eines Präzipitats umfasst;
Isolieren des Präzipitats;
Suspendieren des Präzipitats in einem organischen Lösungsmittel, das ein darin gelöstes Polymer zur Bildung einer Suspension enthält;
Dispergieren der Suspension in der wässrigen Lösung eines Tensids; und
Evaporieren des organischen Lösungsmittels zur Isolierung der Polymermikrosphären.

2. Verfahren nach Anspruch 1, das den zusätzlichen Schritt des Auflösens des Peptidsalzes in einem Minimum an Wasser vor der Neutralisierung des Peptidsalzes umfasst.

3. Verfahren nach Anspruch 2, bei dem das Tensid eines oder mehrere der foldenden ist: Natriumoleat, Natriumstearat, Natriumlaurylsulfat, ein Poly(oxyethylen)sorbitanfettsäureester, Polyvinylpyrrolidin, Polyvinylalkohol, Carboxymethylcellulose, Lecithin, Gelatine oder Hyaluronsäure.

4. Verfahren nach Anspruch 3, bei dem das Tensid Polyvinylalkohol ist und der pH-Wert des Polyvinylalkohols 6,5 bis 7,5 beträgt.

5. Verfahren nach Anspruch 4, bei dem der pH-Wert des Polyvinylalkohols 6,9 bis 7,1 beträgt.

6. Verfahren nach Anspruch 5, bei dem das organische Lösungsmittel Dichloromethan, Chloroform oder Ethylacetat ist.

7. Verfahren nach Anspruch 6, bei dem das organische Lösungsmittel Dichloromethan ist und die Konzentration des Polymers im organischen Lösungsmittel 0,5 bis 30 Gew.-% beträgt.

8. Verfahren nach Anspruch 7, bei dem die Konzentration des Polymers in Dichloromethan 0,5 bis 10 Gew.-% beträgt.

9. Verfahren nach Anspruch 8, bei dem das Peptid Wachstumshormon-freisetzendes Peptid, luteinisiendes Hormon-freisetzendes Hormon, Somatostatin, Bombesin, Gastrin-freisetzendes Peptid, Calcitonin, Bradykinin, Galanin, Melanozyten-stimulierendes Hormon, Wachstumshormon-freisetzender Faktor, Amylin, Tachykinine, Secretin, Parathyreoidhormon, Enkephalin, Endothelin, Calcitonin-Gen-freisetzendes Peptid, Neuromedine, Parathyreoidhormon-verwandtes Protein, Glucagon, Neurotensin, adrenocorticothropes Hormon, Peptid YY, Glucagonfreisetzendes Peptid, vasoaktives intestinales Peptid, Adenylatcyclase-aktivierendes Hypophysenpeptid, Motilin, Substanz P, Neuropeptid Y, oder TSH oder ein Analog oder ein Fragment davon oder ein pharmazeutisch annehmbares Salz davon ist.

10. Verfahren nach Anspruch 9, bei dem das Peptid das LHRH-Analog der Formel pyroGlu-His-Trp-Ser-Tyr-D-Trp-Leu-Arg-Pro-Gly-NH₂ ist.

11. Verfahren nach Anspruch 10, bei dem das Polymer Polylactidco-glycolid, Polycaprolacton oder Polyanhydrid oder ein Copolymer oder Mischungen davon ist.

12. Verfahren nach Anspruch 9, bei dem das Peptid aus der Gruppe von Somatostatin-Analoga, bestehend aus H-D-β-Nal-Cys-Tyr-D-Trp-Lys-Thr-Cys-Thr-NH₂, 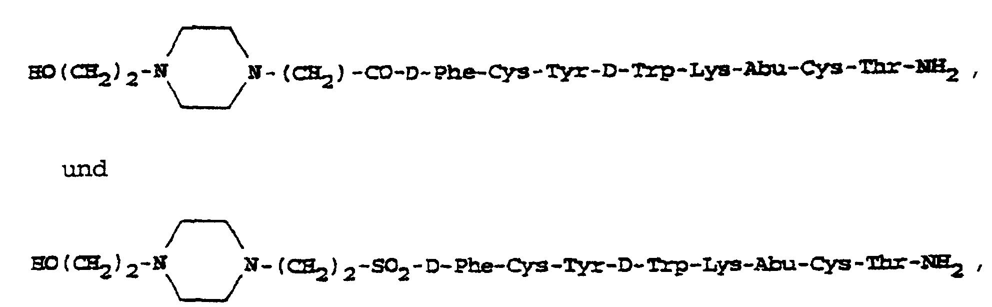 ausgewählt ist.

13. Verfahren nach Anspruch 12, bei dem das Polymer Polylactidco-glycolid, Polycaprolaton oder Polyanhydrid oder ein Copolymer oder Mischungen davon ist.

14. Polymermikrosphäre, die durch das Verfahren nach Anspruch 1 erhältlich ist.

15. Polymermikrosphäre, die durch das Verfahren nach Anspruch 11 erhältlich ist.

16. Polymermikrosphäre, die durch das Verfahren nach Anspruch 13 erhältlich ist.

## Revendications

1. Procédé pour la préparation de microsphères polymères comprenant un polymère et un peptide, qui comprend les étapes de :
neutralisation d'un sel de peptide avec une base faible dans un milieu aqueux dans lequel ledit milieu comprend une suspension d'hydroxyapatite ou une solution de phosphate mono-hydrogéné de calcium pour former un précipité ;
isolation du précipité ;
mise en suspension du précipité dans un solvant organique, qui comprend un polymère dissous dans celui-ci pour former une suspension ;
dispersion de la suspension dans une solution aqueuse d'un surfactant ; et
évaporation du solvant organique pour isoler les microsphères polymères.

2. Procédé selon la revendication 1, comprenant l'étape supplémentaire de dissolution du sel de peptide dans un minimum d'eau avant la neutralisation du sel de peptide.

3. Procédé selon la revendication 2, dans lequel le surfactant est l'un ou plusieurs parmi l'oléate de sodium, le stéarate de sodium, le laurylsulfate de sodium, un ester d'acides gras de poly(oxyéthylène)sorbitan, la polyvinylpyrrolidine, l'alcool polyvinylique, la carboxyméthylcellulose, la lécithine, la gélatine ou l'acide hyaluronique.

4. Procédé selon la revendication 3, dans lequel le surfactant est l'alcool polyvinylique et le pH de l'alcool polyvinylique est de 6,5-7,5.

5. Procédé selon la revendication 4, dans lequel le pH de l'alcool polyvinylique est de 6,9-7,1.

6. Procédé selon la revendication 5, dans lequel le solvant organique est le chlorure de méthylène, le chloroforme ou l'acétate d'éthyle.

7. Procédé selon la revendication 6, dans lequel le solvant organique est le chlorure de méthylène et la concentration du polymère dans le solvant organique est de 0,5 % à 30 % en poids.

8. Procédé selon la revendication 7, dans lequel la concentration du polymère dans le chlorure de méthylène est de 0,5 % à 10 % en poids.

9. Procédé selon la revendication 8, dans lequel le peptide est le peptide de libération de l'hormone de croissance, l'hormone gonadolibérine, la somatostatine, la bombésine, le peptide de libération de la gastrine, la calcitonine, la bradykinine, la galanine, l'hormone mélano-stimuline, le facteur de libération de l'hormone de croissance, *l'amyline,* les tachykinines, la sécrétine, la parathormone, l'encéphaline, l'endothéline, le peptide de libération du gène de la calcitonine, les *neuromédines,* la protéine relative à la parathormone, le glucagon, la neurotensine, l'hormone adrénocorticotrophe, le peptide YY, le peptide de libération du glucagon, le peptide intestinal vasoactif, le peptide d'activation de l'adénylcyclase de l'hypophyse, la *motiline,* la substance P, le neuropeptide Y, ou la TSH ou un analogue ou un fragment de ceux-ci ou un sel pharmaceutiquement acceptable de ceux-ci.

10. Procédé selon la revendication 9, dans lequel le peptide est l'analogue LH-RH selon la formule pyroGlu-His-Trp-Ser-Tyr-D-Trp-Leu-Arg-Pro-Gly-NH₂.

11. Procédé selon la revendication 10, dans lequel le polymère est le polylactide-co-glycolide, la polycaprolactone ou le polyanhydride ou un copolymère ou des mélanges de ceux-ci.

12. Procédé selon la revendication 9, dans lequel le peptide est choisi dans le groupe des analogues de la somatostatine constitué par H-D-β-Nal-Cys-Tyr-D-Trp-Lys-Thr-Cys-Thr-NH₂,

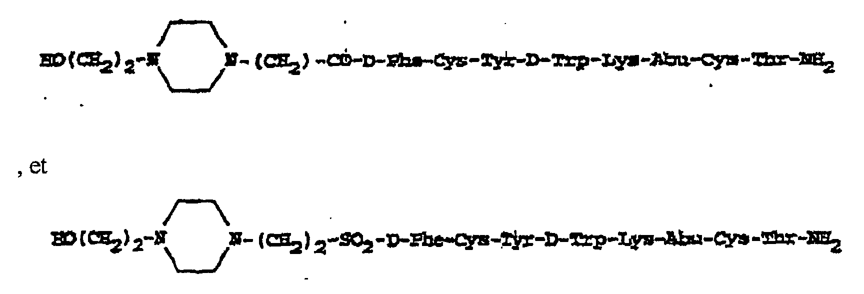

13. Procédé selon la revendication 12, dans lequel le polymère est le polylactide-co-glycolide, la polycaprolactone ou le polyanhydride ou un copolymère ou des mélanges de ceux-ci.

14. Microsphère polymère pouvant être obtenue par le procédé selon la revendication 1.

15. Microsphère polymère pouvant être obtenue par le procédé selon la revendication 11.

16. Microsphère polymère pouvant être obtenue par le procédé selon la revendication 13.
